# EUROPEAN PATENT SPECIFICATION

(11) **EP 0 589 957 B1**
(45) Date of publication and mention of the grant of the patent: **16.10.1996**
(21) Application number: 92911915.4
(22) Date of filing: 18.06.1992
(51) Int. Cl.: A61K 31/415

(54) **USE OF ATIPAMEZOLE FOR THE TREATMENT OF MALE SEXUAL IMPOTENCE**
VERWENDUNG VON ATIPAMEZOL ZUR BEHANDLUNG DER MÄNNLICHEN SEXUELLEN IMPOTENZ
UTILISATION DE L'ATIPAMEZOLE POUR LE TRAITEMENT DE L'IMPUISSANCE SEXUELLE CHEZ L'HOMME

(30) Priority: 18.06.1991 GB 91130773
(43) Date of publication of application: 06.04.1994
(73) Proprietor: ORION-YHTYMÄ OY, SF-02200 Espoo (FI)
(72) Inventor: PERTOVAARA, Antti, SF-02110 Espoo (FI); LINNANKOSKI, Ilkka, SF-00430 Helsinki (FI); VIRTANEN, Raimo, SF-21290 Rusko (FI)
(74) Representative: Sexton, Jane Helen
(86) International application number: FI9200191
(87) International publication number: WO9222296

(56) References cited:
- Drugs Future, vol. 15, no. 5, 1990, "Atipamezole", pages 448-452, see page 449
- Annals of Clinical Research, vol. 20, 1988; E. MacDONALD et al.: "Therapeutic applications of drugs acting on alpha-adrenoceptors", pages 298-310, see abstract; pages 300,302
- PHARMACOLOGY BIOCHEMISTRY AND BEHAVIOR, vol. 42, 1992, Pergamon Press Ltd, US; I. LINNANKOSKI et al.: "Increased sexual behavior in male Macaca arctoides monkeys produced by atipamezole, a selective alpha2-adrenoceptor antagonist", pp. 197-200, see whole document

## Description

This invention relates to use of atipamezole, which is the INN-approved generic name for 4-(2-ethyl-2,3-dihydro-1H-inden-2-yl)-1H-imidazole, or a pharmaceutically acceptable acid addition salt thereof in the manufacture of a medicament for therapeutic treatment of male sexual impotence.

Male impotence is a sexual dysfunction relating to difficulties in achieving and/or maintaining of sufficient penile erection. It can result from a variety of underlying causes ranging from purely psychogenic to completely physical dysfunctioning. Both surgical and pharmacological therapy have been used in the treatment of impotence. Surgical therapy (implantation of a penile prosthetic device) has been used successfully mainly in the case of a purely organic disease. A variety of agents have been suggested for the use as drug therapy in impotence but the reports of their effectiveness are mainly anecdotal in nature. The use of pharmacological therapy in impotence has thus not gained any wide acceptance so far.

A substantial amount of work has been devoted to identifying the neurotransmitters involved in the facilitation and inhibition of male sexual behaviour (see e.g. Bitran and Hull 1987, Neuroscience and Behavioral reviews 11, 365-389). Noradrenergic neuro-transmission seems to have an important role.

MacDonald *et al* (Ann. Clin. Res. 20 298-310 (1988)) indicates that alpha-2-adrenoceptor antagonists may in the future be evaluated for their ability to treat male sexual impotence.

Atipamezole is a selective and potent a₂-adrenoceptor antagonist which is currently marketed for the reversal of sedative-analgesic veterinary drugs. Atipamezole has been disclosed e.g. in the European Patent EP 183492 as useful for the reversal of detomidine.

We have now found that this compound is also very effective in increasing male sexual capacity in a monkey model. These findings suggest that atipamezole would be an effective therapy in male impotence in humans as well.

Another a₂-adrenoreceptor antagonist, yohimbine, is currently used for the treatment of male impotence. Yohimbine increases noradrenergic neurotransmission and has been reported to facilitate the sexual capacity of male animals, although the results of different studies are conflicting. Atipamezole is, however clearly advantageous over yohimbine for this use because of its excellent selectivity. The a₂/a₁ selectivity ratio of atipamezole is 200-300 times higher than that of yohimbine.

### Experimental

Three male and one female stumptail macaques (Macaca Arctoides) were studied in the experiments. The ages of the males were 13, 16 and about 24 years. The age of the female was 6 years.

During the testing period the couple being tested was housed in a single cage (0.6 x 0.9 x 1.2 m) with two compartments. Between the sessions and during the first 10 min of each session a sliding wall separated the male and the female in the test cage. The sliding wall was made of iron bars. The monkeys could see and touch each other through the sliding wall. After the i.m. administration of the studied drug dose/saline control to the male, the observation of the sexual behaviour began as described below. Ten minutes after the drug administration the sliding wall between the male and the female was pulled away, and the observation of sexual activity continued for the next 20 min. At the end of the observation period (=30 min after the drug administration) the sliding wall was replaced. Every time a new couple was being tested, the first three sessions were done as above but without the drug administrations to allow habituation of the couple to each other. These first three sessions were not included in the results.

The time of occurrence and duration of the following behaviour was observed: perineal investigation, mounting, ejaculation, tieing, grooming, direct aggression towards the female, yawns, self-scratching, teeth grinding, shaking of cage and masturbation. In the current report only the number of ejaculations in each session is given, since it gave the most straight forward index of male sexual behaviour. For the same reason, the ejaculations obtained by masturbation and intercourse were pooled in the results.

The experiments were performed once daily seven days a week. Atipamezole was given every other day and saline control during other days. The preliminary results in one monkey indicated that there was no difference in the effect of atipamazole whether it was given every third or other day. Atipamezole doses varied from 0.01 to 0.3 mg/kg (dissolved in saline to get a volume of about 0.2 ml). Each dose was tested 5-15 times in each monkey. Taking into account the saline days, the testing of one dose in one monkey took from 10 days to one month. The order of testing each dose was varied between the monkeys to counterbalance possible serial effects. The difference in the number of ejaculations obtained at a given atipamezole dose and the corresponding saline control days was used as an index of the effect of atipamezole on sexual behaviour. This is how the possible variation in the baseline sexual activity (represented by ejaculations during saline days) could be minimized. The incidence of ejaculations (the percentage of saline days with one or more ejaculations) during saline days was used as an index of baseline sexual activity of each male. One way analysis of variance (ANOVA) and Student's t-test were used in statistical evaluation of the data. P<0.05 was considered to represent a significant difference.

In the saline (=control) conditions the incidences of sexual activity (percentage of sessions with ejaculations produced by copulation and/or masturbation) in the three male monkeys were 7%, 12% and 26%. The oldest male had the lowest and the youngest male the highest incidence of sexual activity in control (=saline) conditions. The sexual activity of the males in saline or atipamezole conditions was not dependent on the estrous cycle of the female.

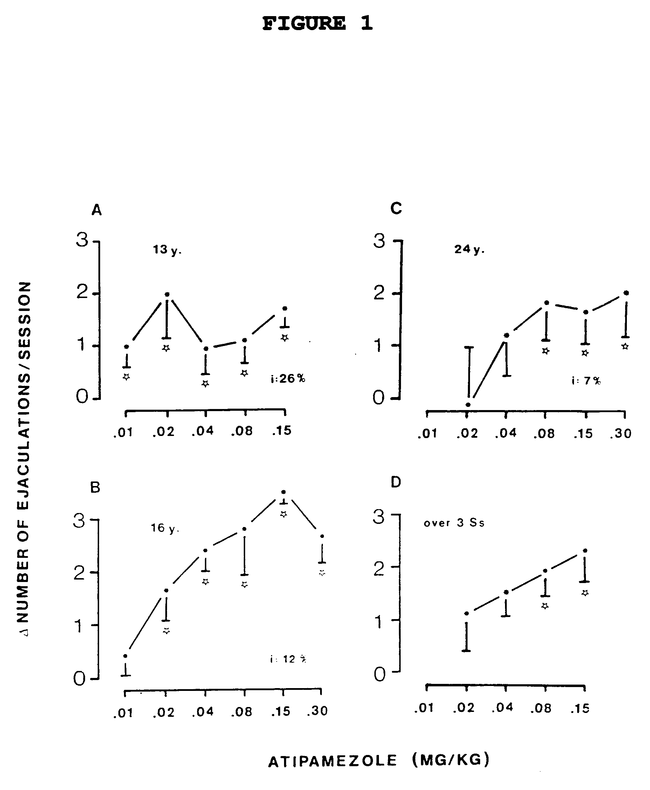

The results obtained with atipamezole are shown in Figure 1 A -D.

Atipamezole increased the number of ejaculations in a dose-dependent way in all three male monkeys (for each individual; p<0.05, ANOVA; Figure 1 A-C). The lowest effective dose of atipamezole varied from 0.01 to 0.08 mg/kg depending on the individual; the younger the male, the lower the lowest effective dose. The average atipamezole-induced increase of ejaculations over the three males also was dose-dependent and significant (p<0.05, ANOVA; Figure 1 D). No other behavioral effects produced by atipamezole were observed except increased alertness.

The drug is preferably administrated perorally, transmucosally, intravenously, intramuscularly or transdermally. The preferable daily dose range is about 0.01 to 1 mg/kg, preferably 0.05 to 0.3 mg/kg for i.v., i.m., transmucosal or transdermal administration and 0.3 to 10 mg/kg for peroral administration.

## Claims

1. Use of atipamezole or a pharmaceutically acceptable acid addition salt thereof in the manufacture of a medicament for the treatment of male sexual impotence.

2. Use according to claim 1 in which the treatment comprises intravenous, intramuscular, transmucosal or transdermal administration of atipamezole or a pharmaceutically acceptable salt thereof in an amount of 0.01 to 1 mg/kg/day.

3. Use according to claim 2 which comprises administration of 0.05 to 0.3 mg/kg/day.

4. Use according to claim 1 in which the treatment comprises peroral administration of atipamezole or a pharmaceutically acceptable salt thereof in an amount of 0.3 to 10 mg/kg/day.

## Patentansprüche

1. Verwendung von Atipamezol oder eines pharmazeutisch verwendbaren Säure-Additions-Salzes davon bei der Herstellung eines Medikaments zur Behandlung von männnlicher sexueller Impotenz.

2. Verwendung nach Anspruch 1, wobei die Behandlung umfaßt: die intravenöse, intramuskuläre, transmukosale oder transdermale Verabreichung von Atipamezol oder eines pharmazeutisch verwendbaren Salzes davon in einer Menge on 0,01 bis 1 mg/kg/Tag.

3. Verwendung nach Anspruch 2, welche die Verabreichung von 0,05 bis 0,3 mg/kg/Tag umfaßt.

4. Verwendung nach Anspruch 1, wobei die Behandlung die perorale Verabreichung von Atipamezol oder eines pharmazeutisch verwendbaren Salzes davon in einer Menge von 0,3 bis 10 mg/kg/Tag umfaßt.

## Revendications

1. Utilisation d'atipamézole ou d'un sel d'addition d'acide de celui-ci, pharmaceutiquement acceptable dans la fabrication d'un médicament pour le traitement de l'impuissance sexuelle chez l'homme.

2. Utilisation selon la revendication 1, dans laquelle le traitement comprend l'administration par voie intraveineuse, intramusculaire, transmuqueuse, ou transdermique d'atipamézole, ou d'un sel de celui-ci, pharmaceutiquement acceptable, en quantité comprise entre 0,01 et 1 mg/kg/jour.

3. Utilisation selon la revendication 2, qui comprend l'administration de 0,05 à 0,3 mg/kg/jour.

4. Utilisation selon la revendication 1, dans laquelle le traitement comprend l'administration par voie orale, d'atipamézole ou d'un sel de celui-ci, pharmaceutiquement acceptable, en quantité comprise entre 0,3 et 10 mg/kg/jour.
